Europäisches Patentamt

European Patent Office

Office européen des brevets

(11) Numéro de publication : **0 094 892**

**B1**

(12) **FASCICULE DE BREVET EUROPÉEN**

(45) Date de publication du fascicule du brevet :
02.01.86

(21) Numéro de dépôt : 83400996.1

(22) Date de dépôt : 18.05.83

(51) Int. Cl.⁴ : **C 12 N 9/74, A 61 K 37/64, A 61 K 37/54**

(54) **Procédé de séparation et de purification des protéases et des antiprotéases de la coagulation sanguine, ainsi que du complexe protéase/antiprotéase.**

(30) Priorité : 19.05.82 FR 8208779

(43) Date de publication de la demande :
23.11.83 Bulletin 83/47

(45) Mention de la délivrance du brevet :
02.01.86 Bulletin 86/01

(84) Etats contractants désignés :
**DE IT NL SE**

(56) Documents cités :
FR-A- 2 154 483
FR-A- 2 261 286
FR-A- 2 461 724

(73) Titulaire : **COMMISSARIAT A L'ENERGIE ATOMIQUE Etablissement de Caractère Scientifique Technique et Industriel**
**31/33, rue de la Fédération**
**F-75015 Paris (FR)**

(72) Inventeur : **Fougnot, Christine**
**139 Rue Gabriel Péri**
**F-93200 Saint Denis (FR)**
Inventeur : **Jozefowicz, Marcel**
**65, 2ème avenue**
**F-60260 Lamorlaye (FR)**
Inventeur : **Rosenberg, Robert D.**
**265 Dean Road**
**Brookline Massachussets 02146 (US)**

(74) Mandataire : **Ores, Irène et al**
**CABINET ORES 6, Avenue de Messine**
**F-75008 Paris (FR)**

Jouve, 18, rue St-Denis, 75001 Paris, France

## Description

La présente invention est relative à un nouveau procédé de séparation et de purification des protéases et des antiprotéases de la coagulation sanguine [et notamment de la thrombine (T) et de l'antithrombine (AT)], ainsi que du complexe thrombine-antithrombine T-AT.

L'étude et la connaissance des mécanismes de la coagulation du sang nécessite de disposer de quantités importantes de thrombine et de l'antithrombine à l'état pur.

Les Demandeurs qui ont acquis une grande expérience dans la préparation des polymères doués de propriétés anticoagulantes (cf. le Brevet français 2 461 724 ou ANN. BIOMED. ENG. 1979, et notamment l'action des inhibiteurs sur l'effet anticoagulant de ces polymères décrits dans ces deux publications), ont mis en évidence des lois d'affinité entre ces polymères et certaines protéines comme les protéases et les antiprotéases de la coagulation sanguine. Aussi, ils se sont fixés pour but de pourvoir à un procédé de séparation et de purification des protéases et des antiprotéases (telles que la T et l'AT) qui soit simple, économique et d'une grande fiabilité.

La présente invention a pour objet un procédé de séparation et de purification des protéases (T) et des antiprotéases (AT) de la coagulation sanguine ainsi que de leur complexe (T-AT), par la mise en contact d'un polymère réticulé insoluble comportant dans sa chaîne le groupe —SO$_3$R$_1$ et/ou le groupe —SO$_2$R$_2$ dans lesquels R$_1$ désigne un atome d'hydrogène ou de métal et R$_2$ désigne le reste d'un acide aminé lié au pont —SO$_2$— par sa fonction amine —NH—, avec une solution contenant les protéases ou les antiprotéases ou leur complexe, la séparation du polymère ayant adsorbé la protéine désirée, le lavage du polymère au tampon, la désorbtion de la protéine adsorbée caractérisé en ce que l'on désorbe la protéine adsorbée par une solution d'un composé polycationique dans le cas de la T ou par une solution d'albumine dans le cas de l'AT ou du complexe T-AT, et on isole la protéine par des moyens connus.

Selon un mode de réalisation avantageux de l'objet de l'invention, le polymère réticulé est le polystyrène.

Selon un autre mode de réalisation avantageux de l'objet de l'invention, le polymère avant sa mise en contact avec les solutions contenant les protéines est équilibré à un pH compris entre 7,3 et 7,5 par lavages successifs avec un tampon approprié.

Conformément à l'invention, le polymère réticulé insoluble mis en œuvre l'est dans la proportion comprise entre 50 et 2 500 mg par mg de protéine présente et, de préférence, entre 50 et 80 mg pour 1 mg de T, entre 200 et 2 000 mg pour 1 mg de AT et entre 220 et 2 000 mg pour 1 mg du complexe T-AT.

Selon un mode de réalisation avantageux de l'objet de l'invention, le composé polycationique est pris dans le groupe qui comprend le bromure d'hexadiméthrine et la polylysine, et la quantité de ce composé mise en œuvre est comprise entre 10 et 15 mg par mg de protéine T.

Selon un autre mode de réalisation avantageux de l'objet de l'invention, la quantité d'albumine mise en œuvre pour la désorption est comprise entre 750 et 2 500 mg par mg de protéine recherchée adsorbée, et de préférence, entre 800 et 1 200 mg pour 1 mg d'AT, et entre 1 000 et 2 000 mg pour 1 mg du complexe T-AT.

Conformément à l'invention, le tampon utilisé pour l'équilibrage du polymère avant l'adsorption et pour le lavage après l'adsorption, est pris dans le groupe qui comprend le tampon Michaelis et le tampon Tris.

Exemples de séparation

Exemple 1

Séparation de la thrombine

1 g de polymère conforme à l'invention est préalablement équilibré à pH 7,4 par plusieurs lavages successifs avec le tampon Tris. On mélange ensuite le polymère pendant une demi-heure à 4 °C avec une solution contenant 18,75 mg de thrombine. Au bout de ce temps, on sépare le polymère (soit par centrifugation, soit sur du verre fritté), et on le lave par 5 fois son volume du même tampon. On mélange ensuite le polymère (pendant une demi-heure à 4 °C) avec 10 ml d'une solution à 10 mg/ml de bromure d'hexadiméthrine. Le polymère complexé avec le bromure d'hexadiméthrine est alors séparé de la solution contenant la thrombine désorbée. Celle-ci est isolée par lyophilisation. On obtient 15 mg de thrombine, soit un rendement de 80 %.

Exemple 2

Séparation de l'antithrombine

10 g de polymère sont préalablement équilibrés à pH 7,4 par plusieurs lavages successifs avec du tampon Michaelis. Le polymère est ensuite mélangé avec 30 ml de plasma pendant une demi-heure à 4 °C. Le gel est ensuite séparé du plasma par centrifugation (ou filtration sur verre fritté). Le polymère est alors lavé avec 5 fois son volume du tampon Michaelis, puis mélangé pendant une demi-heure à 4 °C avec 30 ml de même tampon contenant 100 mg/ml d'albumine (bovine ou humaine). La solution résiduelle contenant l'antithrombine désorbée et l'albumine en excès est alors récupérée par filtration (ou centrifugation). Elle est ensuite injectée sur une colonne d'héparine-sépharose pour séparer les deux protéines. On obtient 3 mg d'antithrombine, soit un rendement de 60 %.

## Exemple 3

### Séparation d'antithrombine sur colonne

La colonne de séparation est préparée avec le polymère préalablement gonflé et équilibré dans le tampon Tris. On fait alors passer le plasma sur le gel de polymère. On élue le plasma non adsorbé par lavage du tampon. On passe alors sur la colonne une solution à 100 mg/ml d'albumine et on récupère la solution contenant l'antithrombine et l'albumine en excès. Cette solution est ensuite passée sur héparine-sépharose comme indiqué dans l'Exemple 2.

On obtient 3 mg d'antithrombine. Rendement : 60 %.

## Exemple 4

### Séparation du T-AT

10 g de polymère sont préalablement équilibrés à pH 7,4 par plusieurs lavages successifs avec du tampon Tris. Le polymère est ensuite mélangé avec 30 ml de plasma contenant le complexe thrombine-antithrombine à raison de 300 nMoles/litre. Après une demi-heure à 4 °C, le polymère est séparé du plasma par centrifugation. Le polymère est alors lavé avec 5 fois son volume du tampon, puis avec 5 volumes du même tampon contenant 100 g/ml d'albumine pendant une demi-heure à 4 °C. Le polymère est alors introduit dans une colonne et le complexe T-AT est élué par du tampon contenant 250 mg/ml d'albumine, jusqu'à l'élution totale (la mesure est effectuée par un radio-immuno essai spécifique). Le complexe est séparé de l'albumine par passage de l'éluat sur une colonne de Concanavaline A-sépharose (ou héparine-sépharose). On récupère 15 mg du complexe, soit un rendement d'environ 50 %.

## Revendications

1. Procédé de séparation et de purification des protéases (T) et des antiprotéases (AT) de la coagulation sanguine ainsi que de leur complexe (T-AT), par la mise en contact d'un polymère réticulé insoluble comportant dans sa chaîne le groupe —SO₃R₁ et/ou le groupe —SO₂R₂— dans lesquels R₁ désigne un atome d'hydrogène ou de métal et R₂ désigne le reste d'un acide aminé lié au pont —SO₂— par sa fonction amine —NH—, avec une solution contenant les protéases ou les antiprotéases ou leur complexe, la séparation du polymère ayant adsorbé la protéine désirée, le lavage du polymère au tampon, la désorption de la protéine adsorbée caractérisé en ce que l'on désorbe la protéine adsorbée par une solution d'un composé polycationique dans le cas de la T ou par une solution d'albumine dans le cas de l'AT ou du complexe T-AT, et on isole la protéine par des moyens connus.

2. Procédé selon la Revendication 1, caractérisé en ce que le polymère réticulé insoluble mis en œuvre, l'est dans la proportion comprise entre 50 et 2 500 mg par mg de protéine présente et, de préférence, entre 50 et 80 mg pour 1 mg de T, entre 200 et 2 000 mg pour 1 mg de AT et entre 220 et 2 000 mg pour 1 mg du complexe T-AT.

3. Procédé selon la Revendication 1, caractérisé en ce que le composé polycationique est pris dans le groupe qui comprend le bromure d'hexadiméthrine et la polylysine et la quantité de ce composé mis en œuvre est comprise entre 10 et 15 mg par mg de protéine T.

4. Procédé selon la Revendication 1, caractérisé en ce que la quantité d'albumine mise en œuvre pour la désorption est comprise entre 750 et 2 500 mg par mg de protéine recherchée adsorbée, et de préférence entre 800 et 1 200 mg pour 1 mg d'AT et entre 1 000 et 2 000 mg pour 1 mg du complexe T-AT.

## Claims

1. A process for the separation and purification of the proteases (T) and antiproteases (AT) involved in blood clotting, and also of their complex (T-AT), by bringing an insoluble crosslinked polymer whose chain contains the group —SO₃R₁ and/or the group —SO₂R₂—, in which R₁ denotes a hydrogen atom or metal atom and R₂ denotes the radical of an amino acid bonded to the —SO₂— bridge by its amine group —NH—, into contact with a solution containing the proteases or antiproteases or their complex, separating off the polymer which has adsorbed the desired protein, washing the polymer with buffer and desorbing the adsorbed protein, characterized in that the adsorbed protein is desorbed by a solution of a polycationic compound in the case of T or by a solution of albumin in the case of AT or the complex T-AT, and the protein is isolated by known means.

2. The process according to Claim 1, characterized in that the insoluble crosslinked polymer is used in a proportion of between 50 and 2 500 mg per mg of protein present, and preferably between 50 and 80 mg per mg of T, between 200 and 2 000 mg per mg of AT and between 220 and 2 000 mg per mg of the complex T-AT.

3. The process according to Claim 1, characterized in that the polycationic compound is taken from the group comprising hexadimethrin bromide and polylysine, and the quantity of this compound used is between 10 and 15 mg per mg of protein T.

4. The process according to Claim 1, characterized in that the quantity of albumin used for desorption is between 750 and 2 500 mg per mg of desired protein adsorbed, and preferably between 800 and 1 200 mg per mg of AT and between 1 000 and 2 000 mg per mg of the complex T-AT.

## Patentansprüche

1. Verfahren zur Abtrennung und Reinigung von Proteasen (T) und von Anti-Proteasen (AT) der Blutgerinnung ebenso wie ihres Komplexes (T-AT) durch Zusammenbringen eines vernetzten unlöslichen Polymeren, das in seiner Kette die $-SO_3R_1$-Gruppe und/oder die $-SO_2R_2$-Gruppe enthält, wobei $R_1$ ein Wasserstoffatom oder Metall bezeichnet und $R_2$ den Rest einer mit der $-SO_2$-Brücke durch ihre $-NH$-Amin-Funktion verbundene Aminosäure bezeichnet, mit einer Proteasen oder Anti-Proteasen oder ihren Komplex enthaltenden Lösung, Abtrennung des Polymeren, das das gewünschte Protein adsorbiert hat, Waschen des Polymeren in Puffer, Desorption des adsorbierten Proteins, dadurch gekennzeichnet, daß man das adsorbierte Protein desorbiert durch eine Lösung mit einer polykationischen Zusammensetzung im Falle von T oder durch eine Lösung von Albumin im Falle von AT oder des Komplexes AT, und man das Protein durch bekannte Mittel isoliert.

2. Verfahren gemäß Anspruch 1, dadurch gekennzeichnet, daß das verwendete unlösliche vernetzte Polymere in einem Anteil von zwischen 50 und 2 500 mg pro mg vorhandenem Protein vorliegt und, vorzugsweise, zwischen 50 und 80 mg pro 1 mg von T, zwischen 200 und 2 000 mg pro 1 mg von AT und zwischen 220 und 2 000 mg pro 1 mg von T-AT-Komplex.

3. Verfahren gemäß Anspruch 1, dadurch gekennzeichnet, daß die polykationische Zusammensetzung genommen ist aus der Gruppe, die das Hexadimethrin-Bromid und das Polylysin umfaßt, und die Menge dieser eingesetzten Zusammensetzung umfaßt zwischen 10 und 15 mg pro mg von T-Protein.

4. Verfahren gemäß Anspruch 1, dadurch gekennzeichnet, daß die für die Desorption eingesetzte Menge Albumin zwischen 750 und 2 500 mg pro mg gesuchtem adsorbiertem Protein umfaßt, und vorzugsweise zwischen 800 und 1 200 mg pro 1 mg von AT und zwischen 1 000 und 2 000 mg pro 1 mg von T-AT-Komplex.